# EUROPÄISCHE PATENTANMELDUNG

(11) **EP 4 600 221 A1**
(43) Veröffentlichungstag der Anmeldung: **13.08.2025**
(21) Anmeldenummer: 25154058.9
(22) Anmeldetag: 27.01.2025
(51) Int. Cl.: C02F 1/467, A47L 11/40, C25B 15/029, C25B 15/08, C25B 9/01, C25B 1/34, C25B 1/04, A61L 2/24, C25B 1/26, A61L 2/03, C02F 103/02

(54) **BEDARFSGESTEUERTE BEREITSTELLUNG EINES REINIGUNGSMITTELS**

(30) Priorität: 08.02.2024 DE 102024201156
(71) Anmelder: BSH Hausgeräte GmbH, 81739 München (DE)
(72) Erfinder: Fremerey, Maximilian, 97633 Saal (DE); Florek, Zenon, 37-100 Lancut (PL)

(57) **Zusammenfassung**

Ein Vorrichtung (105) zur Bereitstellung eines Reinigungsmittels umfasst ein Reaktionsgefäß (125) zur Aufnahme einer wässrigen Lösung (155) eines vorbestimmten Salzes (160); wobei im Reaktionsgefäß (125) zwei Elektroden angebracht sind; eine Gleichspannungsquelle (180) zur Bereitstellung eines elektrischen Stroms durch die Lösung (155) mittels der Elektroden, sodass eine Redox-Reaktion provoziert wird, die das Reinigungsmittel bildet; wobei das Reaktionsgefäß (125) eine gegenüber der Schwerkraft geneigte obere Abdeckung (135) aufweist; und eine Dosiereinrichtung (170) zur Förderung von Reinigungsmittel von einem oberen Bereich der Abdeckung (135) zu einer zu reinigenden Stelle.

## Beschreibung

Die Erfindung betrifft die Bereitstellung von Reinigungsmittel zur Durchführung einer Reinigung in einem Haushalt. Insbesondere betrifft die Erfindung die bedarfsgesteuerte Bereitstellung von Reinigungsmittel an einer zu reinigenden Stelle.

In einem Haushalt wird zur Reinigung einer Bodenfläche Wasser mit einem Reinigungsmittel verwendet. Das Reinigungsmittel soll eine Verunreinigung von der Bodenfläche ablösen, sich mit ihr verbinden und zusammen mit Wasser von der Oberfläche entfernt werden. Das Wasser mit dem Reinigungsmittel kann manuell oder mittels eines automatischen Bodenreinigers auf der Bodenfläche angewandt werden.

Zur Entfernung von Verunreinigungen verfügt das Reinigungsmittel über sogenannte aktive chemische Komponenten. Diese können beispielsweise Tenside, Säuren oder Basen umfassen. Bei korrekter Anwendung und Dosierung kann die Verwendung von Reinigungsmitteln zu guten und hygienischen Reinigungsergebnissen führen. Ein Benutzer kann Reinigungsmittel jedoch auch zu hoch dosieren, so dass unverbrauchtes Reinigungsmittel ins Abwasser gelangen kann und in einem aufwendigen Klärungsprozess daraus wieder entfernt werden muss.

Es wurde vorgeschlagen, Reinigungsmittel erst unmittelbar an dem Ort bereitzustellen, an dem es eingesetzt werden soll. Beispielsweise kann ein Ozongenerator bzw. eine Kaltplasmaquelle aus Luft ein ionisierendes Gas herstellen, welches neben Ozon verschiedene Nitrat- und Nitritverbindungen enthält. Dieses Gas kann in Wasser eingeleitet werden, sodass u. a. geringe Anteile an Wasserstoffperoxid und Salpetersäure gebildet werden. Die so entstehende Reinigungslösung kann effektiv gegen Schmutz wirken und Mikroorganismen inaktivieren.

Bestehende Techniken verwenden relativ langsam ablaufende Prozesse, sodass sie in einem Haushalt kaum praktikabel eingesetzt werden können. Außerdem erlauben viele Ansätze nicht das bedarfsgesteuerte Bereitstellen einer gewünschten Menge oder Konzentration eines Reinigungsmittels. Manche Techniken setzen Wirkstoffe frei, die für eine Person gesundheitsgefährdend sein können.

CN 215272534 U zeigt einen Wassertank für ein Reinigungsgerät, um Wasser und ein Salz miteinander zu mischen. Aus einer entstehenden wässrigen Lösung des Salzes kann mittels Elektrolyse ein Reinigungsmittel hergestellt werden.

Der vorliegenden Erfindung liegt die Aufgabe zugrunde, eine verbesserte Technik anzugeben, um Reinigungsmittel bedarfsgesteuert bereitzustellen. Die Erfindung löst diese Aufgabe mittels der Gegenstände der unabhängigen Ansprüche. Unteransprüche geben bevorzugte Ausführungsformen wieder.

Ein Vorrichtung zur Bereitstellung eines Reinigungsmittels umfasst ein Reaktionsgefäß zur Aufnahme einer wässrigen Lösung eines vorbestimmten Salzes; wobei im Reaktionsgefäß zwei Elektroden angebracht sind; eine Gleichspannungsquelle zur Bereitstellung eines elektrischen Stroms durch die Lösung mittels der Elektroden, sodass eine Redox-Reaktion provoziert wird, die das Reinigungsmittel bildet; wobei das Reaktionsgefäß eine gegenüber der Schwerkraft geneigte obere Abdeckung aufweist; eine Zuführeinrichtung und eine Dosiereinrichtung zur Förderung von Reinigungsmittel von einem oberen Bereich der Abdeckung zu einer zu reinigenden Stelle.

Die Vorrichtung kann dazu eingesetzt werden, das Reinigungsmittel bedarfsgesteuert unmittelbar dort bereitzustellen, wo es benötigt wird. Insbesondere kann die Vorrichtung hierfür in einer Bodendüse oder an einem unteren Gehäuseteil, z.B. eines Reinigungsgeräts, angeordnet sein. Eine Überdosierung von Reinigungsmittel kann vermieden werden. Durch die, insbesondere in einer Gebrauchslage der Bodendüse oder des unteren Gehäuseteils, geneigte obere Abdeckung kann sich Gas, das sich als Zwischen- oder Nebenprodukt der Redox-Reaktion in der Flüssigkeit bilden kann, verbessert dort sammeln, wo das Reinigungsmittel aus dem Reaktionsgefäß entnommen und zu der zu reinigenden Stelle befördert wird. Dadurch kann verhindert werden, dass sich Gase ansammeln und Volumina bzw. Konzentrationen erreichen, die bei einer Freisetzung eine Gesundheitsgefahr für eine Person darstellen können. Das Gas kann in der Flüssigkeit gehalten und zusammen mit ihr zu einem Ausgabebereich geführt werden. Während die Flüssigkeit und das Gas vom Reaktionsgefäß zum einem Ausgabebereich befördert werden, hat das Gas verbessert Gelegenheit, mit dem Reinigungsmittel, dem Wasser oder einer anderen darin enthaltenen Chemikalie zu reagieren. Dadurch kann eine Freisetzung von Gas verringert werden. Eine Gefährdung einer Bedienperson durch das Gas kann reduziert sein. Ein einzuhaltender Grenzwert für das Gas kann leichter eingehalten oder weiter unterschritten werden. Hat das Gas einen Geruch, so kann durch die verringerte Freisetzung des Gases auch eine Geruchsbelastung reduziert sein.

Bevorzugt ist eine Steuervorrichtung zur Steuerung der Dosiereinrichtung in Abhängigkeit einer Anforderung für Reinigungsmittel vorgesehen. Die Anforderung kann durch einen Benutzer der Vorrichtung bereitgestellt werden. So kann ein Reinigungsvorgang punktgenau dann mit der Zugabe von Reinigungsmittel unterstützt werden, wenn dies angefordert wird.

Die Steuervorrichtung kann dazu eingerichtet sein, eine gesteigerte Bereitstellung des Reinigungsmittels gegenüber einer gesteigerten Anforderung um eine vorbestimmte Zeit zu verzögern. Die Anforderung kann einen erhöhten Volumenstrom von Flüssigkeit mit gleichbleibender Konzentration von Reinigungsmittel betreffen. Während der Verzögerung kann die Konzentration von Reinigungsmittel in Flüssigkeit in der Reaktionskammer gesteigert werden. Während der Konzentrationssteigerung kann ein bereitgestellter Volumenstrom konstant gehalten oder verringert sein. Ist die Konzentration des Reinigungsmittels ausreichend erhöht, so kann das höher konzentrierte Reinigungsmittel aus dem Reaktionsgefäß mit dem gewünschten Volumenstrom an der zu reinigenden Stelle bereitgestellt werden.

Die Produktion von Reinigungsmittel kann auch von null aus hochgefahren werden. In einer Lösung ohne Reinigungsmittel können ca. 10 Sekunden vergehen, bis das Reinigungsmittel in einer verwendbaren Konzentration bereitgestellt ist. In einer Ausführungsform kann die Zeit in Abhängigkeit eines Volumenstroms von Flüssigkeit vom Reaktionsgefäß zu einer Ausgabestelle berücksichtigt werden. Optional kann eine Anzeigeeinrichtung vorgesehen sein, um einen Hinweis darauf bereitzustellen, ob bzw. mit welcher Konzentration produziertes Reinigungsmittel bereits vorliegt.

Die Steuervorrichtung kann ferner dazu eingerichtet sein, eine Konzentration von Reinigungsmittel in dem Reaktionsgefäß auf der Basis eines zwischen den Elektroden fließenden Stroms zu bestimmen. Je größer eine Konzentration von Salz zwischen den Elektroden ist, desto mehr Reaktionspartner für die Redox-Reaktion stehen bereit und desto größer kann der durch die wässrige Lösung fließende Strom sein. So kann der fließende Strom einen Hinweis auf den Salzgehalt (die Salinität) in der wässrigen Lösung im Reaktionsgefäß geben. Die bestimmte Konzentration kann beispielsweise mittels einer optischen oder akustischen Anzeige nach außen bereitgestellt werden. Außerdem kann die Steuervorrichtung die bestimmte Konzentration zur Steuerung der restlichen Vorrichtung berücksichtigen.

Es ist weiter bevorzugt, dass die Steuervorrichtung dazu eingerichtet ist, eine Konzentration von Reinigungsmittel in dem Reaktionsgefäß durch Zugabe von Salz in das Reaktionsgefäß zu erhöhen. Die Erhöhung des Salzgehalts kann insbesondere in Abhängigkeit eines bestimmten Salzgehalts erfolgen, der unterhalb eines vorbestimmten Schwellenwerts liegt. Außerdem kann Salz zugegeben werden, falls eine Anforderung für mehr oder höher konzentriertes Reinigungsmittel bestimmt wird.

Zur Zuführung von Salz in das Reaktionsgefäß kann eine Zuführeinrichtung vorgesehen sein. Die Steuervorrichtung ist bevorzugt dazu eingerichtet, die Zuführeinrichtung zu steuern. Weiter bevorzugt ist ein Vorratsbehälter für das Salz vorgesehen. In einer Ausführungsform liegt das Salz in kristalliner Form in dem Vorratsbehälter vor. In einer bevorzugten Ausführungsform liegt das Salz jedoch gelöst in Wasser im Vorratsbehälter vor. Dabei kann die Zuführeinrichtung ein Ventil oder eine Pumpe umfassen. Eine Salinität der Salzlösung im Vorratsbehälter liegt üblicherweise über der Salinität der Flüssigkeit im Reaktionsbehälter. Durch Zuführen der Lösung in den Reaktionsbehälter kann die dortige Salinität angehoben werden.

Wasser und Salz können von einem Benutzer in den Vorratsbehälter eingefüllt werden, sodass sich dort das Salz in dem Wasser löst. In einer Ausführungsform wird so viel Salz in den Vorratsbehälter eingefüllt, bis die Lösung gesättigt ist. Der Salzgehalt der Lösung kann dadurch stets bekannt sein. In einer anderen Ausführungsform ist die Steuervorrichtung dazu eingerichtet, mit einer variablen Salinität der Lösung im Vorratsbehälter zu arbeiten.

Das Ventil kann in einer beliebigen Bauform vorgesehen sein. Aus Gründen der Einfachheit ist bevorzugt, dass das Ventil als Schaltventil ausgeführt ist. In Form einer Pumpe kann die Zuführeinrichtung insbesondere als Verdrängerpumpe ausgeführt sein. Dadurch kann eine Dosierung der Lösung in das Reaktionsgefäß erleichtert sein. Die Pumpe kann als peristaltische Pumpe ausgeführt sein, wobei ein Schlauch vom Vorratsbehälter zum Reaktionsgefäß führt und die peristaltische Pumpe den Schlauch derart verformt, dass die zu fördernde Lösung durch äußere mechanische Verformung des Schlauches durch diesen hindurchgedrückt wird.

Auch die Dosiereinrichtung kann eine Pumpe umfassen, die ebenfalls als peristaltische Pumpe ausgeführt sein kann. Eine peristaltische Pumpe kann einfach aufgebaut sein, ist unempfindlich gegenüber dem zu fördernden Medium und erlaubt es, ein vollständig geschlossenes hydraulisches System zu realisieren. Die peristaltische Pumpe eignet sich zur Dosierung geförderter Flüssigkeit, ist trockenlaufsicher und selbstsperrend. Die Förderung eines Gemisches aus Flüssigkeit und Gas oder Festkörper kann unproblematisch sein. Der Schlauch kann als Verschleißteil ausgelegt und einfach auszutauschen sein.

Die Dosiereinrichtung kann entfallen bzw. integriert mit der Zuführeinrichtung ausgeführt sein. Dabei kann stets so viel Volumen in das Reaktionsgefäß eingebracht wie aus ihm ausgegeben werden. Diese Ausführungsform ist insbesondere vorteilhaft, falls sowohl eine Zufuhr als auch eine Abfuhr in Form von flüssigen Medien erfolgt.

Die Steuervorrichtung ist weiter bevorzugt dazu eingerichtet, die Dosiereinrichtung in Abhängigkeit eines angeforderten Volumenstroms von Reinigungsmittel zu steuern. Gleichzeitig kann die Zuführeinrichtung gesteuert werden, um die Menge von Flüssigkeit, die im Reaktionsgefäß vorliegt, konstant zu halten. Optional kann ein weiterer Vorratsbehälter vorgesehen sein, aus dem eine Übernahme von Wasser in das Reaktionsgefäß gesteuert werden kann. Ein Verhältnis von Wasser zu Salz, die in das Reaktionsgefäß eingefüllt werden, kann in Abhängigkeit eines bereitzustellenden Volumenstroms und einer gewünschten Konzentration von darin gelöstem Reinigungsmittel gesteuert werden.

In einer besonders bevorzugten Ausführungsform können voneinander unabhängige Anforderungen für einen bereitgestellten Volumenstrom von Reinigungsmittel und einer Konzentration des Reinigungsmittels in dem Volumenstrom verarbeitet werden. Die Steuervorrichtung kann dazu eingerichtet sein, die Dosiereinrichtung und die Zugabe von Salz in das Reaktionsgefäß in Abhängigkeit der Anforderung für den Volumenstrom und in Abhängigkeit der Anforderung für die Konzentration sowie des durch die Lösung fließenden Stroms zu steuern.

Auf der Basis des fließenden Stroms kann eine Salinität der Lösung im Reaktionsgefäß bestimmt werden. Die Dosiereinrichtung kann beispielsweise dazu angesteuert werden, den vorbestimmten Volumenstrom von Flüssigkeit aus dem Reaktionsgefäß bereitzustellen. Gleichzeitig kann die Zuführeinrichtung für Salz in das Reaktionsgefäß so gesteuert werden, dass der zwischen den Elektroden fließende Strom einen vorbestimmten Wert erreicht, welcher von der angeforderten Konzentration von Reinigungsmittel in dem bereitgestellten Volumenstrom abhängig ist.

Die Steuerung kann auf klassische Weise mittels eines Regelkreises erfolgen, beispielsweise auf der Basis eines PID-Reglers. In einer anderen Ausführungsform ist ein Kennfeld hinterlegt, welches die Anforderungen und den fließenden Strom auf Steuergrößen für die Dosiereinrichtung und die Zuführeinrichtung abbildet. Das Kennfeld kann verbessert eine Steuerung eines Überganges zwischen unterschiedlichen Kombinationen von Anforderungen erlauben. Das Kennfeld kann einmalig bestimmt und danach an einer Vielzahl baugleicher Vorrichtungen genutzt werden.

Bevorzugt umfasst das Salz Kochsalz. Unter dem Einfluss von Elektrolyse kann das Kochsalz zusammen mit Wasser zu Natronlauge reagieren. Daraus kann in einer zweiten Reaktion zusammen mit Wasser Natriumhypochlorit erzeugt werden. Bei den Reaktionen können Chlorgas und Wasserstoffgas gebildet werden, die im umgebenden Wasser gelöst werden können.

Es ist weiterhin bevorzugt, dass das Reaktionsgefäß werkzeuglos von den restlichen Komponenten getrennt werden kann. Dadurch kann das Reaktionsgefäß leichter ausgetauscht oder gereinigt werden. Eine elektrische Verbindung der Elektroden und fluide Verbindungen für die Zufuhr und die Abfuhr von Flüssigkeit können mittels entsprechender Verbinder getrennt oder wiederhergestellt werden.

Ein Reinigungsgerät zum Einsatz in einem Haushalt umfasst eine hierin beschriebene Vorrichtung. Das Reinigungsgerät kann zur universellen Reinigung unterschiedlicher Oberflächen im Haushalt vorgesehen sein. Insbesondere kann das Reinigungsgerät zur Reinigung eines Bodens vorgesehen sein. Das Reinigungsgerät kann zusätzlich eine mechanische Reinigungsvorrichtung umfassen, beispielsweise eine rotierende Textilwalze oder eine Saugeinrichtung. Ein derartiges Gerät kann als Multi-Use Handstick (MUH) bezeichnet werden. Insbesondere kann die Vorrichtung hierfür in einer Bodendüse oder an einem unteren Gehäuseteil des Reinigungsgeräts angeordnet sein, und so besonders kurze Reaktionsstrecken ermöglichen.

Ein Verfahren zur Bereitstellung eines Reinigungsmittels umfasst Schritte des Erfassens einer Anforderung für Reinigungsmittel; des Steuerns eines elektrischen Stroms durch eine wässrige Lösung eines vorbestimmten Salzes in einem Reaktionsgefäß mit zwei Elektroden, sodass eine Redox-Reaktion provoziert wird, die das Reinigungsmittel bildet; und des Bereitstellens von Reinigungsmittel zusammen mit einem gasförmigen Nebenprodukt der Reaktion an einer zu reinigenden Stelle.

Das Reinigungsmittel kann auf einem Weg vorbestimmter Länge zwischen dem Reaktionsgefäß und der zu reinigenden Stelle geführt werden, wobei in Abhängigkeit eines geförderten Volumenstroms eine vorbestimmte Zeit zur Verfügung steht, dass gelöste Gasbläschen mit der Flüssigkeit in Lösung gehen, bevor die Flüssigkeit mit den Bläschen an der zu reinigenden Stelle ausgegeben wird. Eine Konzentration von freigesetztem Chlor kann dabei sehr gering sein. Die Konzentration kann beispielsweise unterhalb eines Wertes von ca. 0,5 ppm gehalten werden.

Es ist allgemein bevorzugt, dass die Bereitstellung von Reinigungsmitteln als kontinuierliches Verfahren gesteuert wird. Dabei kann eine Menge von Flüssigkeit, die in das Reaktionsgefäß eingebracht wird, einer Menge Flüssigkeit entsprechen, die aus dem Reaktionsgefäß entlassen wird. Das Verfahren kann reaktionsschnell und sicher insbesondere mittels einer hierin beschriebenen Vorrichtung durchgeführt werden. Dazu kann die Vorrichtung eine elektronische Verarbeitungseinrichtung umfassen, die beispielsweise als programmierbarer Mikrocomputer oder Mikrocontroller ausgeführt ist. Das Verfahren kann in Form eines Computerprogrammprodukts mit Programmcodemitteln vorliegen. Das Computerprogrammprodukt kann auch auf einem computerlesbaren Datenträger abgespeichert sein. Merkmale oder Vorteile des Verfahrens können auf die Vorrichtung übertragen werden oder umgekehrt.

Die Erfindung wird nun unter Bezug auf die beiliegenden Figuren genauer beschrieben, in denen:
- Figur 1: ein Haushaltsgerät mit einer Vorrichtung zur Bereitstellung von Reinigungsmittel; und
- Figur 2: ein Ablaufdiagramm eines Verfahrens; darstellt.

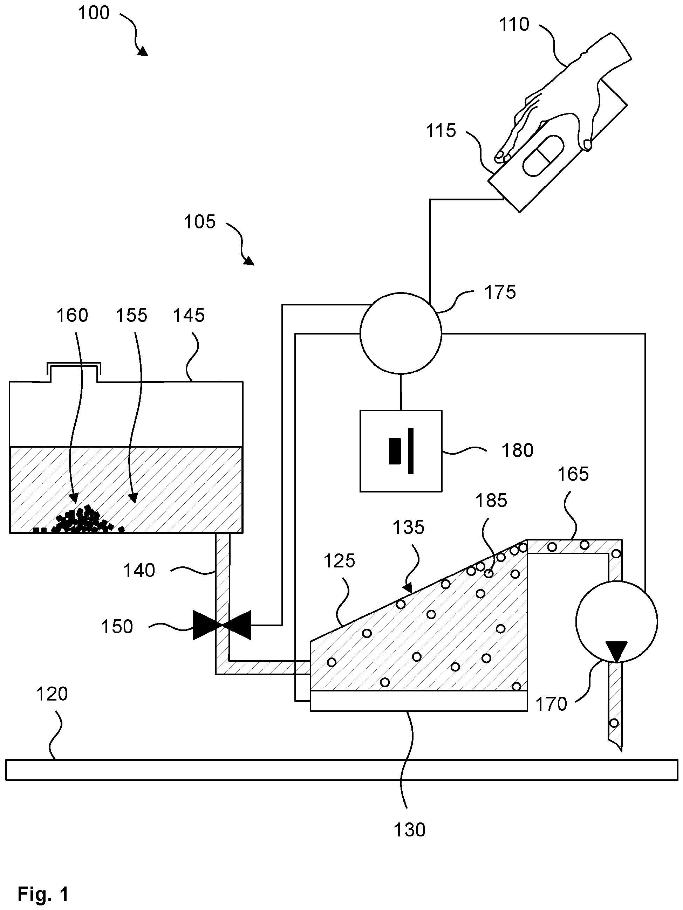

Figur 1 zeigt ein Haushaltsgerät 100 mit einer Vorrichtung 105 zur Bereitstellung von Reinigungsmittel. Das Haushaltsgerät 100 ist bevorzugt als handgeführter Bodenreiniger mit Stiel (Multi-Use Handstick, MUH) ausgeführt. Eine Person 110 kann das Haushaltsgerät 100 an einem Griff 115 am oberen Ende des Stiels greifen und das Gerät am unteren Ende des Stiels über eine vorbestimmte Oberfläche, beispielsweise eine Bodenfläche 120, führen. Obwohl nicht in Figur 1 dargestellt, kann das Haushaltsgerät 100 eine klassische Einrichtung zur Bearbeitung der Oberfläche umfassen, beispielsweise eine rotierende Textilwalze, eine Saugvorrichtung oder eine Kehrvorrichtung.

Die hierin beschriebene Vorrichtung 105 kann zum Einsatz in unterschiedlichen Haushaltsgeräten 100 geeignet sein, die auch beispielsweise ein handgehaltenes Gerät ohne Stiel oder ein autonomes Reinigungsgerät, insbesondere für die Bodenfläche 120, umfassen können.

Die Vorrichtung 105 umfasst ein Reaktionsgefäß 125, in welchem ein elektrolytischer Reaktor 130 vorgesehen ist. Der Reaktor 130 umfasst zwei Elektroden, zwischen denen eine Spannung angelegt werden kann, um einen elektrischen Strom durch eine Lösung zwischen den Elektroden zu bewirken. Je größer Oberflächen der Elektroden sind, desto stärker kann eine Reaktion sein, die elektrolytisch bewirkt wird. Die Elektroden können beispielsweise als Flächen oder Gitter ausgeführt sein, und ein Abstand zwischen ihnen ist bevorzugt konstant.

In der dargestellten Ausführungsform liegt der Reaktor 130 beispielhaft flach an einem bezüglich der Schwerkraft unteren Ende des Reaktionsgefäßes 125, in einer anderen Ausführungsform kann der Reaktor 130 auch gegenüber der Schwerkraft geneigt sein. Ein oberes Ende des Reaktionsgefäßes 125 ist durch eine Abdeckung 135 gebildet, die gegenüber der Schwerkraft geneigt ist. In einem Bereich des Reaktionsgefäßes 125, der bevorzugt unterhalb der Abdeckung 125 liegt, ist ein Zufluss 140 für Flüssigkeit aus einem Vorratsbehälter 145 vorgesehen. Im Zufluss 140 kann eine steuerbare Zuführeinrichtung 150 vorgesehen sein, mit der dosiert werden kann, welcher Volumenstrom aus dem Vorratsbehälter 145 in das Reaktionsgefäß 125 eingelassen wird. Im Vorratsbehälter 145 befindet sich eine Lösung 155 eines vorbestimmten Salzes 160 in Wasser. Wasser und Salz können bei Bedarf manuell in den Vorratsbehälter 145 eingefüllt werden, beispielsweise durch die Person 110.

An einem oberen Bereich des Reaktionsgefäßes 125 ist ein Abfluss 165 vorgesehen, von dem aus ein Inhalt des Reaktionsgefäßes 125 an einer zu reinigenden Stelle ausgegeben werden kann. Zur Förderung und/oder Dosierung von Flüssigkeit aus dem Reaktionsgefäß 125 zu dieser Stelle kann eine Dosiereinrichtung 170 vorgesehen sein, die bevorzugt als Verdrängerpumpe ausgeführt ist. Insbesondere kann eine Schlauchpumpe bzw. eine peristaltische Pumpe eingesetzt werden.

Eine Steuervorrichtung 175 ist dazu eingerichtet, einen elektrischen Strom aus einem Energiespeicher 180 durch die Elektroden des Reaktors 130 zu steuern. Dabei kann zwischen den Elektroden eine vorbestimmte Spannung anliegen, die bevorzugt unter ca. 12 V, weiter bevorzugt unter ca. 5 V, liegt. In Abhängigkeit weiterer Parameter kann in einer beispielhaften Ausführungsform der zwischen den Elektroden fließende Strom unterhalb von ca. 1000 mA betragen. Der Energiespeicher 180 ist bevorzugt in Form eines Akkumulators ausgeführt.

Die Steuervorrichtung 175 ist dazu eingerichtet, mittels der Zuführeinrichtung 150 in Wasser gelöstes Salz in den Reaktionsbehälter 125 einzulassen. Der Reaktionsbehälter 125 kann vollständig mit Flüssigkeit gefüllt sein. Wird nun eine Spannung an die Elektroden des Reaktors 130 angelegt, so kann eine elektrolytische Reaktion provoziert werden, die letztlich Reinigungsmittel im Reaktionsgefäß 125 bereitstellen kann. Dabei können insbesondere folgende Reaktionen ablaufen:

*2NaCl* + 2*H*₂*O* → *2NaOH* + H*₂* + *Cl*₂

*2NaOH* + *Cl*₂ → *NaOCl* + *NaCl* + *H*₂*O*

Mittels der ersten Reaktion kann Natronlauge bereitgestellt werden, die bereits als Reinigungsmittel genutzt werden kann. Mittels der zweiten Reaktion kann die Natronlauge in Natriumhypochlorit und Kochsalz weiter umgewandelt werden. Natriumhypochlorit (NaOCl) ist auch als Natronbleichlauge bekannt und kann als Reinigungsmittel eingesetzt werden. NaOCl hat gute Eigenschaften zur Reinigung und Bindung von Schmutz und kann inaktivierend auf Mikroorganismen wirken.

Bei den Reaktionen freiwerdendes Chlorgas und freiwerdender Wasserstoff können zumindest teilweise im umgebenden Wasser als ClO⁻ bzw. H₃O⁺ in Lösung gehen. Gasförmig austretendes Chlor- bzw. Wasserstoffgas kann sich in Bläschen 185 sammeln, die in der Flüssigkeit aufsteigen können, bis sie die obere Abdeckung 135 erreichen.

Die obere Abdeckung 135 ist derart geneigt, dass die Bläschen 185 dazu gezwungen werden, auf einem vorbestimmten Pfad an der Abdeckung 135 entlang aufzusteigen. Dadurch können die Bläschen 185 in Richtung des Abflusses 165 gelenkt werden, wo sie zusammen mit der umgebenden Flüssigkeit in den Abfluss 165 eintreten können. Die Dosiereinrichtung 170 befördert Flüssigkeit und Bläschen 185 zu einem Auslass, wo beide an einer zu reinigenden Stelle freigesetzt werden können. Während ihres Transports durch den Abfluss 165 können sich die Bläschen 185 teilweise oder vollständig in der umgebenden Flüssigkeit lösen, sodass letztlich nur sehr geringe Mengen bzw. Konzentrationen von Gas im Bereich der Bodenfläche 120 ausgegeben werden können. Ausgegebenes Gas kann vorteilhaft dazu genutzt werden, Mikroorganismen im Bereich der zu reinigenden Stelle zu inaktivieren.

Die obere Abdeckung 135 erstreckt sich in einer Richtung, die gegenüber der Schwerkraft geneigt ist. Ist die Abdeckung 135 eben, so kann sie einen spitzen Winkel mit der Richtung der Schwerkraft einschließen. Je kleiner der Winkel ist, desto größer kann eine Kraft sein, mit der die Bläschen 185 an der Abdeckung 135 entlang befördert werden können. Dieser Zusammenhang gilt im Wesentlichen auch, wenn die Abdeckung 135 nicht eben, sondern beispielsweise konkav oder konvex gekrümmt ist. Weitere mögliche Formen umfassen beispielsweise einen Kegel oder eine Pyramide.

Bevorzugt ist der Abfluss 165 in einem Bereich am Reaktionsgefäß 125 angeschlossen, wo die Abdeckung 135 ihren höchsten Punkt hat. Die obere Abdeckung 135 ist ferner so geformt, dass Bläschen 185 von allen Punkten durch ihre Auftriebskraft in Richtung des Abflusses 165 geführt werden. Von jedem Punkt an der unteren Oberfläche der Abdeckung 135 kann ein vertikaler Aufstieg eines Bläschens 185 mit einer horizontalen Bewegung in Richtung des Abflusses 165 gekoppelt sein. So kann das Bilden einer Gasblase abseits des Abflusses 165 im Reaktionsgefäß 125 vermieden werden. Es ist zu beachten, dass diese Bedingung bevorzugt auch dann erfüllt ist, wenn das Haushaltsgerät 100 während seiner Handhabung geneigt oder geschwenkt wird.

In der dargestellten Ausführungsform sind die Elektroden im Reaktor 130 übereinander angeordnet, sodass über den gesamten Bereich des Reaktors 130 sowohl Chlor- als auch Wasserstoffbläschen aufsteigen können, die bis zur oberen Abdeckung 135 aufsteigen, daran entlanglaufen und in den Abfluss 165 eintreten können.

In einer anderen Ausführungsform können die Elektroden nebeneinander liegen. Dabei können unterschiedliche Abschnitte der oberen Abdeckung 135 über den Elektroden liegen, sodass die Gase separiert werden können. Beispielsweise können die Abschnitte in unterschiedlichen Richtungen geneigt sein oder zwischen den Abschnitten kann ein Trennelement vorgesehen sein, um dem Übertritt von Bläschen 185 zwischen den Abschnitten zu verhindern. Die Abdeckung 135 kann in den Abschnitten in unterschiedlichen Richtungen geneigt sein. Über der einen Elektrode kann auf diese Weise Chlorgas und über der anderen Elektrode Wasserstoffgas gesammelt werden. Gesammelte Gas kann jeweils in der beschriebenen Weise abgeführt werden, wobei zwei Abflüsse 165 vorgesehen sein können. Alternativ kann eines der Gase auch anders abgeführt werden, beispielsweise in einen Filter oder zu einer Substanz, mit der das Gas reagieren kann.

Der Reaktor 135 kann wie dargestellt horizontal ausgerichtet sein oder auch in einem vorbestimmten Winkel gegenüber der Schwerkraft. In einer Ausführungsform sind der Reaktor 130 und die obere Abdeckung 135 in einem im Wesentlichen konstanten Abstand zueinander angebracht, sodass sie im Wesentlichen parallel zueinander liegen. Dabei kann der Reaktor 130 mit demselben Winkel gegenüber der Schwerkraft geneigt sein wie die obere Abdeckung 135.

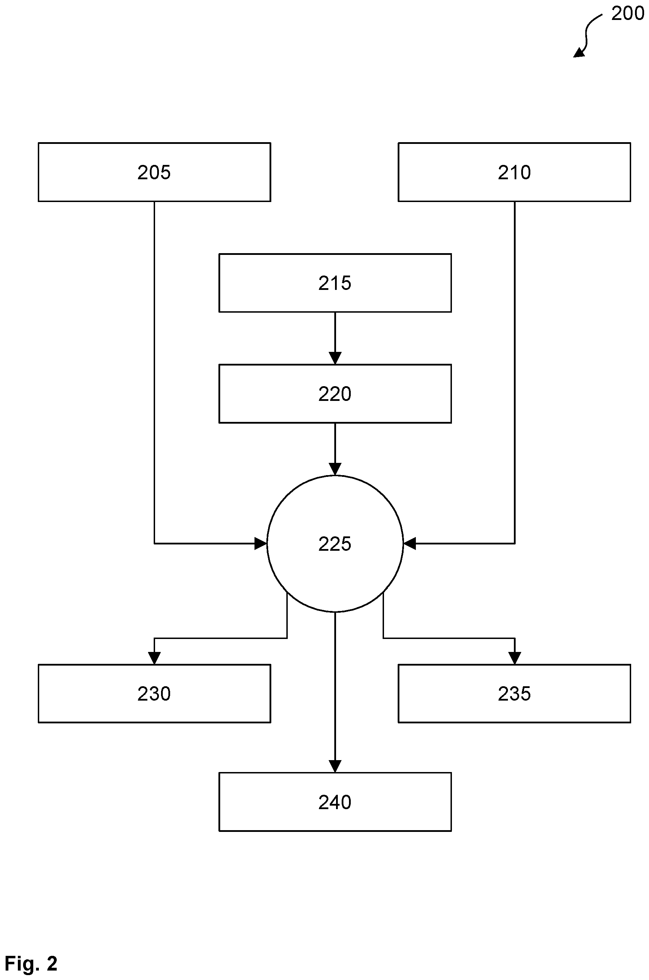

Figur 2 zeigt ein Ablaufdiagramm eines Verfahrens 200 zur Bereitstellung von Reinigungsmittel. Das Verfahren 200 kann insbesondere mittels einer Vorrichtung 105 ausgeführt werden.

In einem Schritt 205 kann eine Anforderung für eine Konzentration von Reinigungsmittel in ausgegebener Flüssigkeit erfasst werden. Die Anforderung kann durch eine Person 110 bereitgestellt sein, welche die Vorrichtung 105 bzw. ein sie umgebendes Haushaltsgerät 100 bedient.

In einem Schritt 210 kann in entsprechender Weise eine Anforderung für einen auszugebenden Volumenstrom von Flüssigkeit erfasst werden. Die zweite Anforderung kann ebenfalls durch die Person 110 bereitgestellt werden. Es ist bevorzugt, dass beide Anforderungen unabhängig voneinander geändert werden können.

In einem Schritt 215 kann ein elektrischer Strom durch das Reaktionsgefäß gesteuert werden, um mittels einer elektrolytischen Redox-Reaktion in Wasser gelöstes Salz in Reinigungsmittel umzuwandeln. Dazu kann eine vorbestimmte Gleichspannung auf der Basis von Energie aus dem Energiespeicher 180 erzeugt und an Elektroden des Reaktors 130 angelegt werden.

In einem Schritt 220 kann bestimmt werden, wie stark ein zwischen den Elektroden fließender Strom ist. Dazu kann insbesondere ein Strom bestimmt werden, der zwischen dem Energiespeicher 180 und einer der Elektroden des Reaktors 130 fließt. Je größer der Strom ist, desto stärker kann eine Umwandlungsreaktion sein, die Reinigungsmittel im Reaktionsgefäß 125 bereitstellt.

In einem Schritt 225 können die bestimmten Parameter verarbeitet werden. In einer Ausführungsform kann auf der Basis des bestimmten Stroms eine Konzentration von reaktionsfähigen Substanzen im Bereich der Elektroden des Reaktors 130 bestimmt werden. Diese Substanzen können insbesondere in Wasser gelöstes Kochsalz umfassen. Die bestimmte Konzentration kann an die Person 110 ausgegeben werden. Außerdem kann eine weitere Verarbeitung auf der Basis der bestimmten Konzentration erfolgen.

Im Rahmen der Verarbeitung kann bestimmt werden, welche Mengen bzw. Volumenströme an Wasser und Salz in das Reaktionsgefäß 125 eingefüllt werden sollen. Ein aus dem Reaktionsgefäß 125 bereitgestellter Volumenstrom kann gleich groß sein. Eine Konzentration von Reinigungsmittel im Reaktionsgefäß kann in Abhängigkeit eines fließenden Stroms bestimmt werden. Zur Erhöhung der Konzentration des Reinigungsmittels kann die Salinität im Reaktionsgefäß 125 durch verstärkte Zugabe von Salz erhöht werden. Zur Verringerung der Konzentration kann verstärkt Wasser eingefüllt werden.

In einem Schritt 230 kann eine Zugabe von Salz aus dem Vorratsbehälter 145 in das Reaktionsgefäß 125 gesteuert werden. Durch die Zugabe von Salz kann die Umwandlungsreaktion in Reinigungsmittel beschleunigt werden. Gleichzeitig kann in einem Schritt 235 eine Entnahme von Flüssigkeit mit Reinigungsmittel aus dem Reaktionsgefäß 125 gesteuert werden. Die Entnahme kann dosiert erfolgen, insbesondere mittels der Dosiereinrichtung 170. In einem optionalen Schritt 240 kann eine Zugabe von Wasser in das Reaktionsgefäß 125 gesteuert werden. So kann dafür gesorgt werden, dass das Reaktionsgefäß 125 stets vollständig mit gleich viel Flüssigkeit gefüllt ist und der bereitgestellte Volumenstrom unabhängig von der bereitgestellten Konzentration des Reinigungsmittels ist.

### Bezugszeichen

- 100: Haushaltsgerät
- 105: Vorrichtung
- 110: Person
- 115: Griff
- 120: Bodenfläche

- 125: Reaktionsgefäß
- 130: Reaktor
- 135: obere Abdeckung
- 140: Zufluss
- 145: Vorratsbehälter
- 150: Zuführeinrichtung
- 155: Lösung
- 160: Salz
- 165: Abfluss
- 170: Dosiereinrichtung
- 175: Steuervorrichtung
- 180: Energiespeicher
- 185: Bläschen

- 200: Verfahren
- 205: Anforderung Konzentration erfassen
- 210: Anforderung Volumenstrom erfassen
- 215: elektrischen Strom durch das Reaktionsgefäß steuern
- 220: fließenden Strom bestimmen
- 225: Parameter verarbeiten
- 230: Zugabe von Salz steuern
- 235: Entnahme von Reinigungsmittel steuern
- 240: Zugabe von Wasser steuern

## Patentansprüche

1. Vorrichtung (105) zur Bereitstellung eines Reinigungsmittels, wobei die Vorrichtung (105) folgende Elemente umfasst:
- ein Reaktionsgefäß (125) zur Aufnahme einer wässrigen Lösung (155) eines vorbestimmten Salzes (160);
- wobei im Reaktionsgefäß (125) zwei Elektroden angebracht sind;
- eine Gleichspannungsquelle (180) zur Bereitstellung eines elektrischen Stroms durch die Lösung (155) mittels der Elektroden, sodass eine Redox-Reaktion provoziert wird, die das Reinigungsmittel bildet;
- wobei das Reaktionsgefäß (125) eine gegenüber der Schwerkraft geneigte obere Abdeckung (135) aufweist; und
- eine Dosiereinrichtung (170) zur Förderung von Reinigungsmittel von einem oberen Bereich der Abdeckung (135) zu einer zu reinigenden Stelle.

2. Vorrichtung (105) nach Anspruch 1, ferner umfassend eine Steuervorrichtung (175) zur Steuerung der Dosiereinrichtung (170) in Abhängigkeit einer Anforderung für Reinigungsmittel.

3. Vorrichtung (105) nach Anspruch 2, wobei die Steuervorrichtung (175) dazu eingerichtet ist, eine gesteigerte Bereitstellung des Reinigungsmittels gegenüber einer gesteigerten Anforderung um eine vorbestimmte Zeit zu verzögern.

4. Vorrichtung (105) nach einem der Ansprüche 2 bis 3, wobei die Steuervorrichtung (175) dazu eingerichtet ist, eine Konzentration von Reinigungsmittel in dem Reaktionsgefäß (125) auf der Basis eines zwischen den Elektroden fließenden Stroms zu bestimmen.

5. Vorrichtung (105) nach einem der Ansprüche 2 bis 4, wobei die Steuervorrichtung (175) dazu eingerichtet ist, eine Konzentration von Reinigungsmittel in dem Reaktionsgefäß (125) durch Zugabe von Salz (160) in das Reaktionsgefäß (125) zu erhöhen.

6. Vorrichtung (105) nach einem der vorangehenden Ansprüche, ferner umfassend eine Zuführeinrichtung (150) für Salz (160) in das Reaktionsgefäß (125); wobei die Steuereinrichtung dazu eingerichtet ist, die Zuführeinrichtung (150) zu steuern.

7. Vorrichtung (105) nach Anspruch 6, ferner umfassend einen Vorratsbehälter mit Salz (160) in gelöster Form; wobei die Zuführeinrichtung (150) ein Ventil oder eine Pumpe umfasst.

8. Vorrichtung (105) nach einem der Ansprüche 2 bis 7, wobei die Steuervorrichtung (175) dazu eingerichtet ist, die Dosiereinrichtung (170) in Abhängigkeit eines angeforderten Volumenstroms von Reinigungsmittel zu steuern.

9. Vorrichtung (105) nach einem der Ansprüche 2 bis 8, wobei die Steuervorrichtung (175) dazu eingerichtet ist, die Dosiereinrichtung (170) und die Zugabe von Salz (160) in das Reaktionsgefäß (125) in Abhängigkeit einer ersten Anforderung für einen Volumenstrom von Reinigungsmittel und einer zweiten Anforderung einer Konzentration des Reinigungsmittels sowie eines durch die Lösung (155) fließenden Stroms zu steuern.

10. Vorrichtung (105) nach Anspruch 9, wobei die Steuerung mittels eines Kennfelds erfolgt.

11. Vorrichtung (105) nach einem der vorangehenden Ansprüche, wobei das Salz (160) Kochsalz umfasst.

12. Vorrichtung (105) nach einem der vorangehenden Ansprüche, wobei das Reaktionsgefäß (125) werkzeuglos von den restlichen Komponenten getrennt werden kann.

13. Reinigungsgerät (100) zum Einsatz in einem Haushalt, umfassend eine Vorrichtung (105) nach einem der vorangehenden Ansprüche.

14. Verfahren (200) zur Bereitstellung eines Reinigungsmittels, wobei das Verfahren folgende Schritte umfasst:
- Erfassen (205, 210) einer Anforderung für Reinigungsmittel;
- Steuern (215) eines elektrischen Stroms durch eine wässrige Lösung (155) eines vorbestimmten Salzes (160) in einem Reaktionsgefäß (125) mit zwei Elektroden, sodass eine Redox-Reaktion provoziert wird, die das Reinigungsmittel bildet; und
- Bereitstellen (235) von Reinigungsmittel zusammen mit einem gasförmigen Nebenprodukt der Reaktion an einer zu reinigenden Stelle.

15. Verfahren (200) nach Anspruch 14, wobei die Bereitstellung von Reinigungsmittel als kontinuierliches Verfahren gesteuert wird.
